# EUROPÄISCHE PATENTSCHRIFT

(11) **EP 1 771 659 B1**
(45) Veröffentlichungstag und Bekanntmachung des Hinweises auf die Patenterteilung: **14.05.2008**
(21) Anmeldenummer: 05759709.8
(22) Anmeldetag: 24.06.2005
(51) Int. Cl.: F04B 33/00, F04B 45/02, F04B 45/04, A61F 2/78, A61F 2/80

(54) **PUMPE MIT EINER BEWEGBAREN WANDUNG UND VERWENDUNG EINER DERARTIGEN PUMPE**
PUMP COMPRISING A MOVING WALL AND USE OF A PUMP OF THIS TYPE
POMPE AYANT UNE PAROI MOBILE ET UTILISATION D'UNE TELLE POMPE

(30) Priorität: 28.07.2004 DE 102004036669
(43) Veröffentlichungstag der Anmeldung: 11.04.2007
(73) Patentinhaber: Otto Bock HealthCare IP GmbH & Co. KG, 37115 Duderstadt (DE)
(72) Erfinder: MOSLER, Lüder, 37115 Duderstadt (DE); HILLMANN, Martin, 37115 Duderstadt (DE); CARSTENS, Ralf, 37083 Göttingen (DE)
(74) Vertreter: Lins, Edgar
(86) Internationale Anmeldenummer: PCT/DE2005/001124
(87) Internationale Veröffentlichungsnummer: WO 2006/012820

(56) Entgegenhaltungen:
- WO-A-02/067825
- DE-A1- 1 952 065
- DE-C- 745 981
- FR-A- 2 538 350
- US-A- 3 133 696
- US-A- 6 004 116

## Beschreibung

Die Erfindung betrifft eine Pumpe mit einer ein abgeschlossenes Fluidvolumen bildenden Wandung, die mittels einer ersten externen Kraft in Richtung Volumenverkleinerung und mittels einer zweiten Kraft - nach einer vorhergehenden Volumenverkleinerung - in Richtung Volumenvergrößerung bewegbar ist, und mit einem Einlassventil, das mit einer Einlassöffnung kommuniziert, und mit einem Auslassventil in einer Auslassleitung des Fluidvolumens.

Die Erfindung betrifft ferner eine Verwendung einer derartigen Pumpe.

Derartige Pumpen sind beispielsweise als Schlauchpumpen bekannt, bei denen aus einem mit einem Fluid gefüllten Schlauch das Fluid mittels den Schlauch zusammenpressender und in eine Längsrichtung des Schlauches bewegter Druckrollen ausgepresst wird. Durch die Bewegung der Rolle wird das stromwärts liegende Ende des Schlauches mit Fluid wieder gefüllt, wenn dieses Ende mit einem Fluidvorrat in Verbindung steht. Die Antriebskraft für die Bewegung der Rollen wird durch einen Motor erzeugt, der beispielsweise als elektrischer oder hydraulischer Motor ausgebildet sein kann. Derartige Pumpen werden zur Förderung eines Volumens in Druckrichtung verwendet.

Eine andere Art der Pumpe der eingangs erwähnten Art sind Membranpumpen, bei denen das Fluidvolumen durch die mittels eines Pleuels hin- und herbewegte Membran verkleinert und anschließend wieder vergrößert wird. Das Pleuel überträgt somit sowohl die erste Kraft zur Volumenverkleinerung als auch die zweite Kraft zur Volumenvergrößerung.

In vielen Fällen ist es lediglich erforderlich, eine Pumpleistung nur in bestimmten Betriebszuständen abzurufen, wenn in einer Vorrichtung Bewegungen ablaufen, deren Kraftfluss für die Betätigung einer Pumpe verwendbar ist. So ist es beispielsweise bekannt, den Zwischenraum zwischen einem Amputationsstumpf eines Patienten und einem darüber angeordneten, luftdicht ausgebildeten Liner zu evakuieren, um durch das gebildete Vakuum einen festen Sitz des mit einer Prothese verbundenen Liners zu gewährleisten. Hierfür wird eine Kolbenpumpe verwendet, die beim Auftreten des Patienten mittels der Prothese auf den Boden einen Evakuierungshub ausübt und mittels einer Rückstellfeder zurückgestellt wird. Derartige Pumpen sind, insbesondere wegen des erforderlichen Rückstellmechanismus, relativ voluminös.

Eine derartige Pumpe ist durch WO 02/067825 A2 als Teil einer Beinprothese bekannt. Ein zylindrisches Gehäuse der Pumpe ist mit dem Schaft einer Prothese verbunden. In dem Gehäuse ist ein mit einer Unterschenkelprothese verbundener Zylinder abgedichtet angeordnet. In dem Zylinder befindet sich wiederum ein Kolben, der fest mit dem Gehäuse - und damit mit dem Schaft der Prothese - verbunden ist. Durch diese Anordnung werden drei Kammern gebildet, nämlich zwischen der Oberseite des Gehäuses und der Oberseite des in dem Gehäuse abgedichtet bewegbaren Zylinders, zwischen der Oberseite des (fest mit dem Gehäuse verbundenen) Kolbens und der Oberseite des Zylinders sowie zwischen der Unterseite des Kolbens und der Unterseite des Zylinders, wobei der letztgenannte Raum bei einer Gewichtsbelastung eine stoßdämpfende Funktion ausübt. Bei einer Gewichtsbelastung der Prothese über den Schaft bewegt sich der Zylinder in dem Gehäuse nach oben, wodurch Luft aus einem abgeschlossenen Raum des Schaftes durch die Vergrößerung des Raumes zwischen der Oberseite des Kolbens und der oberen Wand des Zylinders eingesaugt wird. Gleichzeitig verkleinert sich der Raum zwischen der oberen Wand des Zylinders und der oberen Wand des Gehäuses. Die verdrängte Luft kann ins Freie entweichen. Entfällt die Belastung durch das Körpergewicht, stellt sich die Pumpe durch eine den Zylinder an seiner Unterseite nach oben drückenden Rückstellfeder nach oben, wobei die in der unteren Kammer stoßdämpfend komprimierte Luft die Rückstellbewegung unterstützt. Die bekannte Vakuumpumpe besteht aus zahlreichen, gegeneinander abzudichtenden Bauteilen sowie einer Rückstellfeder, die in dem Gehäuse untergebracht werden müssen, das dadurch ein Mindestvolumen nicht unterschreiten kann.

Der Erfindung liegt daher die Aufgabe zugrunde, eine Pumpe der eingangs erwähnten Art so auszubilden, dass sie auf kleinem Raum ausführbar ist.

Zur Lösung dieser Aufgabe ist erfindungsgemäß eine Pumpe mit den Merkmalen des Patentanspruchs 1 ausgebildet.

Bei der erfindungsgemäßen Pumpe wird der Arbeitshub, mit dem das Fluid, insbesondere Luft, aus einem abgeschlossenen Volumen abgesaugt wird, durch die Rückstellkraft des elastisch verformbaren Materials bewirkt. Die vorherige Verformung zur Volumenverkleinerung des Fluidmaterials erfolgt durch eine extern angreifende erste Kraft. Die erfindungsgemäße Pumpe ermöglicht somit eine sehr unkomplizierte und kleinvolumige Ausführung, mit der ein geringer bis mittlerer Unterdruck erzeugt werden kann.

In einer ersten bevorzugten Ausführungsform der Erfindung weist die Wandung zwei starre, einander gegenüberliegende Wände auf, wobei das elastisch verformbare Material in dem durch die Wände gebildeten Zwischenraum angeordnet ist. Hierbei kann das elastisch verformbare Material mit einem am Rand umlaufenden und das Fluidvolumen begrenzenden Dichteinsatz gebildet sein. Diese Ausführungsform bietet den Vorteil, dass die externe Kraft unmittelbar an einer der starren Wände angreifen kann.

In einer anderen, ebenfalls Vorteile aufweisenden Ausführungsform ist die Wandung flexibel ausgebildet, wobei an der flexiblen Wandung das elastisch verformbare Material, vorzugsweise flächig, anliegt. Dabei kann mit einer relativ dünnen Materiallage eine für viele Anwendungsfälle ausreichend hohe Rückstellkraft erzeugt werden.

Das elastische Material kann ein offenporiger Schaumstoff sein, der innerhalb des Fluidvolumens angeordnet ist und der die Rückstellkraft nach einer durchgeführten Volumenverkleinerung ausübt. Demgemäß wird der Schaumstoff vom Fluid, das bevorzugt Luft ist, durchströmt. In dieser Ausführungsform nimmt die Erzeugung der Rückstellkraft überhaupt keinen zusätzlichen Raum ein, da hierfür das Fluidvolumen selbst ausgenutzt wird. Dabei ist es zweckmäßig, wenn der Schaumstoff das Fluidvolumen - bis auf konstruktive Restvolumina - vollständig ausfüllt. Ein alternatives durchströmbares elastisches Material, das für die Erfindung verwendbar ist, ist ein Abstandsgewirk.

Die Realisierung der erfindungsgemäßen Pumpe gelingt in einfacher Weise, wenn das elastische Material allseitig von der flexiblen Wandung umgeben ist. Möglich ist es aber auch, die Wandung teilweise starr auszubilden und einen solchen Teil der Wandung flexibel zu gestalten, der für die Volumenverkleinerung benötigt wird.

Die erfindungsgemäße Pumpe lässt sich vorzugsweise mit einer bevorzugten flächigen Ausdehnung mit einer demgegenüber geringen Dicke erstellen und so in vielen Fällen unproblematisch in eine Vorrichtungskonstruktion integrieren.

Zum Zusammenpressen des Fluidvolumens ist wenigstens ein flächig an der flexiblen Wandung anliegendes Andruckelement vorgesehen. Insbesondere kann das Fluidvolumen mit der flexiblen Wandung zwischen zwei flächigen Andruckelementen angeordnet sein.

Die Anordnung der Ventile kann auf den entsprechenden schmalen Seiten der flexiblen Wandung erfolgen, bevorzugt jedoch auch in Ausnehmungen eines der Andruckelemente oder beider Andruckelemente, wodurch Walkvorgänge der flexiblen Wandung verringert werden.

Die erfindungsgemäße Pumpe lässt sich mit Vorteil im Kraftfluss eines Systems integrieren, in dem Kräfte entstehen, die zur Ausübung einer der beiden Kräfte ausgenutzt werden. Die erfindungsgemäße Pumpe ist insbesondere als Vakuumpumpe geeignet.

In einem speziellen Anwendungsfall stellt die Pumpe einen Teil einer Prothese für eine untere Extremität dar. Vorzugsweise wird dabei die beim Auftreten aufgrund des Körpergewichts entstehende Kraft als erste Kraft ausgenutzt. Die Pumpe kann insbesondere zur Vakuumunterstützung eines Saugschaftes der Prothese, insbesondere zur Evakuierung des Zwischenraums zwischen einem Liner und dem Prothesenschaft eingesetzt werden. Ein bevorzugter Einsatzort für die erfindungsgemäße Pumpe ist ein künstlicher Fuß, der die flächige Ausbildung der Pumpe senkrecht zu dem bei der Belastung mit dem Körpergewicht entstehenden Kraftfluss besonders gut ermöglicht.

Die Erfindung soll im Folgenden anhand von in der Zeichnung dargestellten Ausführungsbeispielen näher erläutert werden. Es zeigen:
- Figur 1: eine schematische Darstellung einer Pumpe im Ausgangszustand nach einer ersten Ausführungsform;
- Figur 2: die Pumpe gemäß Figur 1 im zusammengedrückten Zustand;
- Figur 3: eine Pumpe in einem Ausgangszustand nach einer zweiten Ausführungsform;
- Figur 4: eine Pumpe in einer dritten Ausführungsform, die in einen künstlichen Fuß integriert ist;
- Figur 5: eine Anordnung eines elastisch verformbaren Materials, bestehend aus zwei Lagen mit zueinander zeigenden Profilierungen, die im unbelasteten Zustand einen Fluidraum bilden;
- Figur 6: die Anordnung gemäß Figur 5 im belasteten Zustand, in dem das komprimierte Material der Lage den Fluidraum vollständig ausfüllt;
- Figur 7: eine Ausführungsform einer erfindungsgemäßen Pumpe mit zwei starren, einander gegenüber liegenden Platten und einem am Rand umlaufenden Einsatz aus einem elastisch verformbaren Material, im unbelasteten Zustand;
- Figur 8: die Anordnung gemäß Figur 7 im belasteten Zustand.

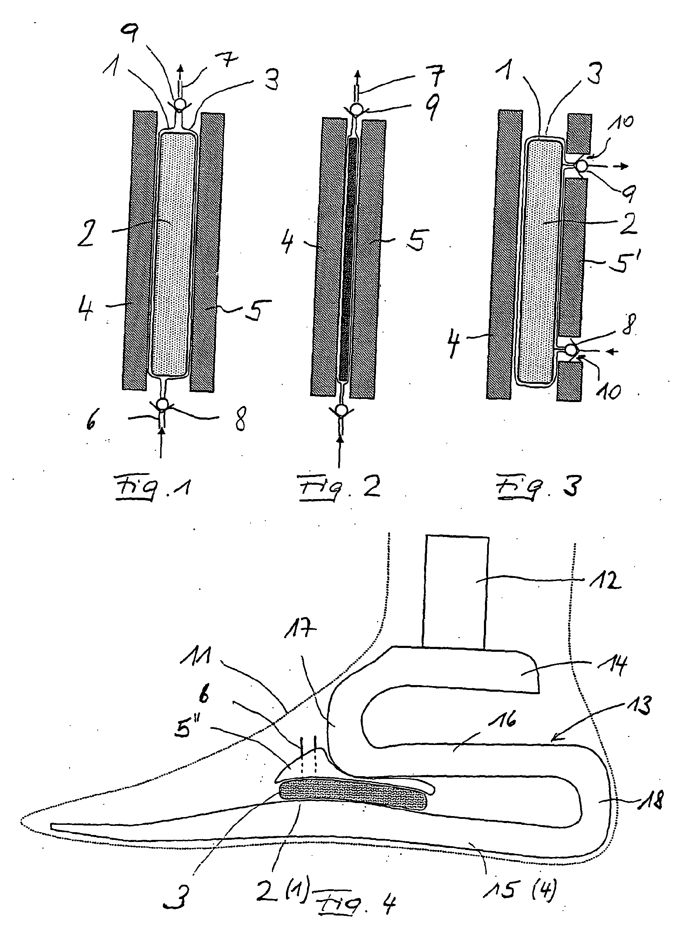

Die in Figur 1 dargestellte Pumpe weist ein geschlossenes Fluidvolumen 1 auf, das praktisch vollständig mit einem elastischen Material in Form eines offenporigen, elastischen Schaumstoffs 2 ausgefüllt ist. Der Schaumstoff 2 weist eine im Wesentlichen flächige Ausdehnung mit einer geringen Breite auf. Er ist allseitig von einer flexiblen Wandung 3 umgeben, die somit vier schmale Seiten und zwei demgegenüber groß dimensionierte flächige Seiten aufweist. An den flächigen Seiten liegen zwei flächige Andruckelemente 4, 5 an.

An gegenüberliegenden schmalen Seiten weist die flexible Wandung 3 eine rohr- oder schlauchförmige Zuführung 6 und einen rohr- oder schlauchförmigen Ausgang 7 auf. Sowohl die Zuführung 6 als auch der Ausgang 7 sind mit jeweils einem Rückschlagventil 8, 9 versehen.

Für den Pumpvorgang werden die Andruckelemente 4, 5 relativ aufeinander zu bewegt, wie dies Figur 2 verdeutlicht. Dadurch wird das Fluidvolumen 1 mit dem darin befindlichen Schaumstoff 2 zusammengedrückt, wodurch Fluid, vorzugsweise Luft, über das Rückschlagventil 9 und den Ausgang 7 entweicht. Entfällt die Andruckkraft der Andruckelemente 4, 5, sorgt die elastische Rückstellkraft des Schaumstoffs 2 für die Rückstellung des Fluidvolumens 1 in den Ausgangszustand der Figur 1, wobei Fluid über den Zugang 6 und das Rückschlagventil 8 in das Fluidvolumen 1 eingesaugt wird.

Bei der in Figur 3 dargestellten Modifikation der Pumpe ist die Andruckplatte 5' mit Ausnehmungen 10 versehen, in denen die Rückschlagventile 8, 9 angeordnet sind. Dadurch werden die Walkvorgänge der flexiblen Wandung 3 im Bereich der Rückschlagventile 8, 9 deutlich reduziert.

Figur 4 zeigt ein Anwendungsbeispiel für eine Pumpe der in den Figuren 1 bis 3 beschriebenen Art. Die Pumpe ist dabei in einen künstlichen Fuß 11 integriert, dessen Funktionsaufbau mit einem Unterschenkelrohr 12 einer Unterschenkelprothese verbunden ist. Der Funktionsteil des künstlichen Fußes besteht aus einem S-förmigen Federeinsatz 13, dessen freie Enden einen oberen Anschlussschenkel 14 und einen unteren Sohlenschenkel 15 ausbilden. Dazwischen befindet sich ein im Wesentlichen horizontales Zwischenstück 16, das mit dem Anschlussschenkel 14 bzw. dem Sohlenschenkel 15 durch jeweils ein gekrümmtes Übergangsstück 17, 18 so verbunden ist, dass das im Wesentlichen horizontale Zwischenstück 16 unter Gewichtseinwirkung relativ zum Sohlenschenkel 15 des Einsatzes 13 federn kann. Unter Einwirkung des Gewichts des Prothesenträgers beim Aufsetzen des Fußes auf den Boden wird somit der Abstand zwischen dem Zwischenstück 16 und dem Sohlenschenkel 15 verringert. Diese Abstandsverringerung wird für die erfindungsgemäß eingesetzte Pumpe ausgenutzt, indem das Zwischenstück 16 mit einem Andruckelement 5" verbunden ist. Das an sich flächige Andruckelement 5" ist in seiner Form an die Form des Zwischenstücks 16 und des gekrümmten Übergangs 17 angepasst, um die Positionierung des Andruckelements 5" zu erleichtern. Zwischen dem Andruckelement 5" und dem hier als Gegen-Andruckelement 4 fungierenden Sohlenschenkel 15 befindet sich das durch den Schaumstoff 2 ausgefüllte Fluidvolumen 1 innerhalb der den Schaumstoff 2 allseitig umgebenden flexiblen Wandung 3. Nur schematisch ist einer der Anschlüsse 6, 7 angedeutet, der sich durch eine Ausnehmung 10 des Andruckelements 5" erstreckt und beispielsweise als Zugang 6 über eine Schlauchleitung mit dem Zwischenraum zwischen einem Amputationsstumpf und einem den Amputationsstumpf umgebenden Liner verbunden sein kann, um diesen Zwischenraum zu evakuieren.

Die Funktion der in den Fuß 11 eingesetzten Pumpe entspricht vollständig der anhand der Figuren 1 bis 3 erläuterten Funktion. Als Andruckkraft für das Andruckelement 5" wirkt die Belastung des künstlichen Fußes 11 mit dem Körpergewicht, wodurch das Zwischenstück 16 nach unten in Richtung auf den Sohlenschenkel 15 gedrückt wird und so das Fluidvolumen 1 und den Schaumstoff 2 zusammenpresst. Dadurch entweicht Luft aus dem Fluidvolumen 1. Bei der Entlastung des künstlichen Fußes 11, spätestens beim Abheben des Fußes 11 vom Boden für den nächsten Schritt, bewirkt der Schaumstoff 2 die Rückstellung der Pumpe in die Figur 4 dargestellte Ausgangsstellung. Dabei wird über den Zugang 6 Luft aus dem Zwischenraum zwischen dem Amputationsstumpf und dem umgebenden Liner in das Fluidvolumen 1 eingesaugt, also in dem Zwischenraum ein gewünschter Unterdruck erzeugt, der den Sitz des Liners am Amputationsstumpf stabilisiert.

Es ist erkennbar, dass ein künstlicher Fuß 11 für die Integration der Pumpe gemäß Figur 4 geeignet ist, weil eine flächige Ausbildung des Fluidvolumens 1 und des Schaumstoffs 2 senkrecht zum (vertikalen) Kraftfluss aufgrund der ebenfalls zur Flächigkeit tendierenden Anatomie des Fußes 11 gut möglich ist. Dennoch ist es selbstverständlich auch möglich, eine entsprechende Pumpe in anderen, eine relative Bewegung zueinander ausführenden Prothesenteilen, beispielsweise in einem Kniegelenk, anzuordnen.

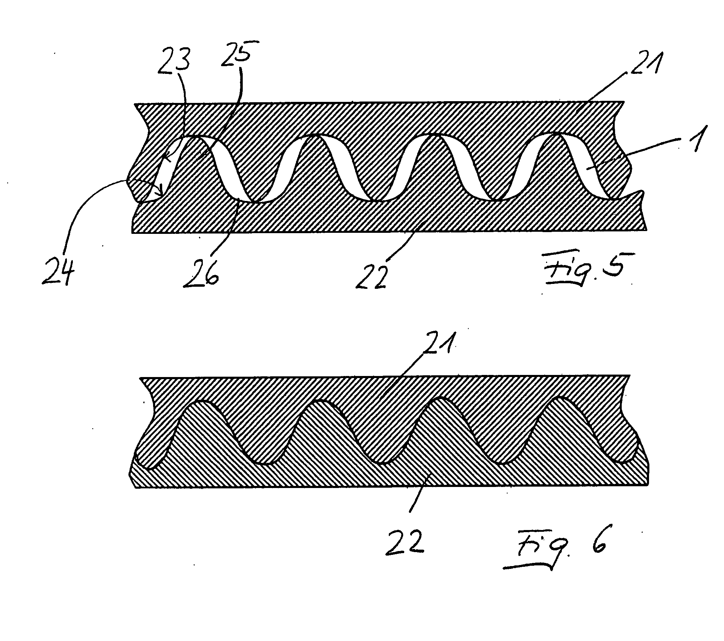

Die Figuren 5 und 6 zeigen ein Ausführungsbeispiel, bei dem das elastisch verformbare Material durch zwei Lagen 21, 22 gebildet ist, die mit jeweils einer profilierten Oberfläche 23, 24 zueinander zeigen. Die profilierten Oberflächen sind dabei durch Vorsprünge 25 gebildet, deren Breite kleiner ist als eine Ausnehmung 26 zwischen den Vorsprüngen 25. Demgemäß ragen die Spitzen 25 der Lage 21 in die Ausnehmungen 26 der Lage 22 und umgekehrt, wobei die den Fluidraum 1 bildenden, von dem elastisch verformbaren Material nicht ausgefüllten Räume entstehen.

Wird nun auf die Lagen 21, 22 ein das Zusammendrücken bewirkender Druck ausgeübt, werden die Spitzen 25 in der Höhenrichtung zusammengedrückt und das Material weicht in die Breite aus, sodass die Räume zwischen den Spitzen 25 und den Ausnehmungen 26 - im Idealfall vollständig - ausgefüllt werden, wie dies in Figur 6 dargestellt ist. Entfällt die externe Kraft, die die Lagen 21, 22 gegeneinander drückt, stellt sich die Ausgangssituation der Lagen 21, 22 ein, wie sie in Figur 5 dargestellt ist. Die Rückstellung bewirkt ein Ansaugen von Fluid, insbesondere Luft, aus einem mit dem Fluidraum 1 verbundenen Unterdruckraum.

Die Lagen 21, 22 können aus einem so stabilen Kunststoffmaterial gebildet sein, dass die Lagen 21, 22 zugleich die Wandung 3 bilden. Alternativ können die Lagen 21, 22 jedoch auch mit Andruckplatten 4, 5, wie sie in den Ausführungsformen gemäß der Figuren 1 bis 3 dargestellt sind, zusammenwirken.

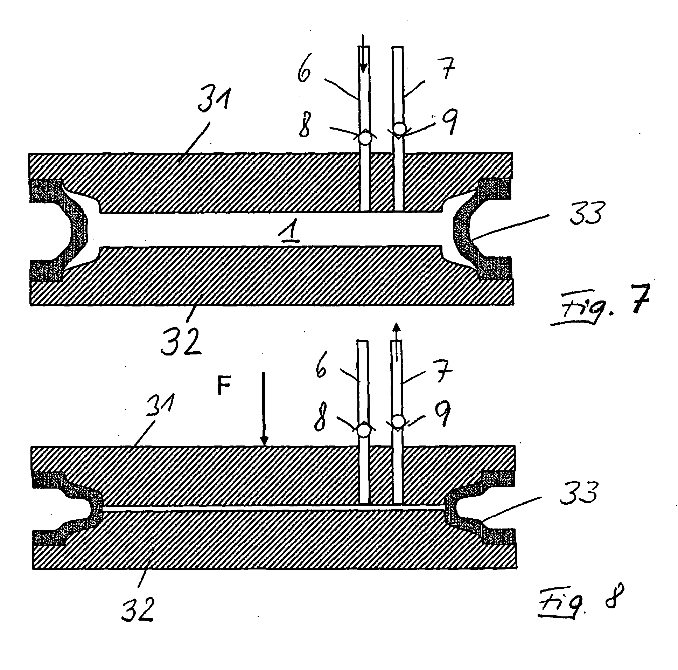

Bei dem in den Figuren 7 und 8 dargestellten Ausführungsbeispiel wird der Fuidraum durch zwei zueinander parallele starre Wände 31, 32 und durch einen umlaufenden, die Ränder der starren Wände 31, 32 miteinander abdichtend verbindenden Einsatz 33 aus einem elastisch verformbaren Material begrenzt. Der Einsatz 33 ist dabei ein hohlkehlartiges Profil aus einem stabilen, fluiddichten Material, das mit den Wänden 31, 32 durch Klebung o.ä. fluiddicht verbunden ist.

Durch eine externe erste Kraft F werden die starren Wände 31, 32 gegeneinander gedrückt, wie dies in Figur 8 zum Ausdruck gebracht ist. Dadurch wird der Fluidraum 1 maximal verkleinert, wodurch das in dem Fluidraum 1 enthaltene Fluid durch den Ausgang 7 entweicht. Entfällt die externe Kraft F, stellt sich der in Figur 7 dargestellte Zustand aufgrund der Rückstellkraft des Einsatzes 33 wieder ein, wodurch über die Zuführung 6 Fluid in den Fluidraum 1 gesaugt wird.

Es ist erkennbar, dass die in den Figuren 5 bis 8 dargestellten Ausführungsbeispiele in gleicher Weise eingesetzt werden können, wie die Ausführungsbeispiele gemäß den Figuren 1 bis 3, also insbesondere auch in einem künstlichen Fuß o. ä.

Die erfindungsgemäßen Pumpen können insbesondere in Prothesenteilen auch für andere Zwecke verwendet werden, beispielsweise als Hydraulikpumpe für die Steuerung von dynamischen Funktionen, beispielsweise für die Steuerung von hydraulischen Dämpfungszylindern oder zur Bewegung von konstruktiven Elementen der Prothese, beispielsweise aus einem nicht gekoppelten in einen gekoppelten Zustand, um eine dynamische Anpassung an die Gebrauchssituation vorzunehmen.

## Patentansprüche

1. Pumpe, gebildet durch eine ein abgeschlossenes Fluidvolumen (1) bildenden Wandung (3; 21, 22; 31, 32, 33), die mittels einer ersten Kraft (F) in Richtung Volumenverkleinerung und mittels einer zweiten Kraft - nach einer vorhergehenden Volumenverkleinerung - in Richtung Volumenvergrößerung bewegbar ist, durch ein Einlassventil (8), das mit einer Einlassöffnung (6) kommuniziert und durch ein Auslassventil (9) in einer Auslassleitung (7) des Fluidvolumens (1), als Teil eines Systems, in dem Kräfte, insbesondere durch ein Körpergewicht entstehen, wobei die Pumpe als Unterdruckpumpe ausgebildet ist, deren Volumen durch die im System entstehende Kraft als die erste Kraft gegen ein elastisch verformbares Material (2; 21, 22, 33) verkleinerbar ist, wobei das Fluid aus dem Fluidvolumen (1) über die Auslassleitung (7) entweicht und das elastisch verformbare Material (2; 21, 22; 33) durch die Wandung (3; 21, 22; 31, 32, 33) selbst gebildet oder innerhalb des Fluidvolumens (1) angeordnet ist, durch seine Rückstellbewegung nach Beendigung der ersten Kraft die gegen den hergestellten Unterdruck wirkende zweite Kraft bildet.

2. Pumpe nach Anspruch 1, **dadurch gekennzeichnet, dass** die Wandung zwei starre, einander gegenüberliegende Wände (31, 32) aufweist und dass das elastisch verformbare Material (33) in dem durch die Wände gebildeten Zwischenraum angeordnet ist.

3. Pumpe nach Anspruch 2, **dadurch gekennzeichnet, dass** das elastisch verformbare Material mit einem Rand umlaufenden und das Fluidvolumen (1) begrenzenden Dichteinsatz (33) gebildet ist.

4. Pumpe nach Anspruch 1, **dadurch gekennzeichnet, dass** die Wandung (3) flexibel ausgebildet ist und dass an der flexiblen Wandung (3) das elastisch verformbare Material (2) anliegt.

5. Pumpe nach Anspruch 4 **dadurch gekennzeichnet, dass** das elastische Material ein durchströmbares Material ist, das innerhalb des Fluidvolumens (1) angeordnet ist und dessen Rückstellkraft die zweite Kraft bildet.

6. Pumpe nach Anspruch 5, **dadurch gekennzeichnet, dass** das durchströmbare material ein offenporiger Schaumstoff (2) ist.

7. Pumpe nach Anspruch 6, **dadurch gekennzeichnet, dass** das elastische Material das Fluidvolumen (1) ausfüllt.

8. Pumpe nach Anspruch 7, **dadurch gekennzeichnet, dass** das elastische Material allseitig von der flexiblen Wandung (3) umgeben ist.

9. Pumpe nach einem der Ansprüche 4 bis 8, **dadurch gekennzeichnet, dass** das elastische Material mit einer flächigen Ausdehnung und mit einer gegenüber der flächigen Ausdehnung kleinen Dicke ausgebildet ist.

10. Pumpe nach einem der Ansprüche 1 bis 9, **dadurch gekennzeichnet, dass** das elastisch verformbare Material zwei aneinander anliegende Lagen (21, 22) mit zueinander zeigenden, Zwischenräume bildenden Profilierungen (23, 24) aufweist und dass die Zwischenräume durch die erste Kraft verkleinerbar sind.

11. Pumpe nach Anspruch 14, **dadurch gekennzeichnet, dass** die Zwischenräume zwischen den beiden Lagen (21, 22) durch die externe erste Kraft auf Null verkleinerbar sind.

12. Pumpe nach einem der Ansprüche 4 bis 11, **gekennzeichnet durch** wenigstens ein flächig an der flexiblen Wandung (3) anliegenden Druckelement (4, 5, 5', 5").

13. Pumpe nach einem der Ansprüche 1 bis 12, **dadurch gekennzeichnet, dass** sich die Ventile (8, 9) auf den der Dicke entsprechenden schmalen Seiten der Wandung (3) befinden.

14. Pumpe nach Anspruch 13, **dadurch gekennzeichnet, dass** die Ventile (8, 9) an gegenüber liegenden schmalen Seiten der Wandung (3) angeordnet sind.

15. Pumpe nach Anspruch 12, **dadurch gekennzeichnet, dass** die Ventile (8, 9) in Ausnehmungen (10) der Andruckelemente (5', 5") angeordnet sind.

16. Pumpe nach Anspruch 15, **dadurch gekennzeichnet, dass** die Ventile (8, 9) in Ausnehmungen (10) eines der Andruckelemente (5', 5") angeordnet sind.

17. Verwendung einer Pumpe nach einem der Ansprüche 1 bis 16 als Teil einer Prothese (11) für eine untere Extremität.

18. Verwendung einer Pumpe nach Anspruch 17, bei der die beim Auftreten aufgrund des Körpergewichts entstehende Kraft als erste Kraft (F) benutzt wird.

19. Verwendung nach Anspruch 17 oder 18 zur Vakuumunterstützung eines Saugschaftes der Prothese (11).

## Claims

1. Pump formed by a wall (3; 21, 22; 31, 32, 33) which forms a closed-off fluid volume (1) and which can be moved by means of a first force (F) in the direction of a volume reduction and by means of a second force, after a preceding volume reduction, in the direction of a volume increase, an inlet valve (8) which communicates with an inlet port (6) and an outlet valve (9) in an outlet line (7) of the fluid volume (1), as part of a system, in which forces are generated, in particular by a body weight, the pump being designed as a vacuum pump, the volume of which can be reduced by means of the force generated in the system as the external first force against an elastically deformable material (2; 21, 22; 33), the fluid from the fluid volume (1) escaping over the outlet line (7) and the elastically deformable material (2; 21, 22, 33) being formed by the wall (3; 21, 22; 31, 32, 33) itself or being arranged within the fluid volume (1), forming via its return force motion, after the termination of the external force, the second force acting counter to the generated vacuum.

2. Pump according to Claim 1, **characterized in that** the wall has two rigid walls (31, 32) lying opposite one another, and **in that** the elastically deformable material (33) is arranged in the interspace formed by the walls.

3. Pump according to Claim 2, **characterized in that** the elastically deformable material is formed by a sealing insert (33) running around at the edge and delimiting the fluid volume (1).

4. Pump according to Claim 1, **characterized in that** the wall (3) is designed flexibly, and **in that** the elastically deformable material (2) bears against the flexible wall (3).

5. Pump according to Claim 4, **characterized in that** the elastic material is a material which is capable of throughflow and is arranged within the fluid volume (1) and the return force of which forms the second force.

6. Pump according to Claim 5, **characterized in that** the material capable of throughflow is an open-pored foam (2).

7. Pump according to Claim 6, **characterized in that** the elastic material fills the fluid volume (1).

8. Pump according to Claim 7, **characterized in that** the elastic material is surrounded on all sides by the flexible wall (3).

9. Pump according to one of Claims 4 to 8, **characterized in that** the elastic material is designed with a large-area extent and with a thickness which is small, as compared with the large-area extent.

10. Pump according to one of Claims 1 to 9, **characterized in that** the elastically deformable material has two layers (21, 22) bearing against one another and having profilings (23, 24) pointing toward one another and forming interspaces, and **in that** the interspaces can be reduced by means of the first force.

11. Pump according to Claim 10, **characterized in that** the interspaces between the two layers (21, 22) can be reduced to zero by means of the external first force.

12. Pump according to one of Claims 1 to 11, **characterized by** at least one pressure element (4, 5, 5', 5") bearing against the flexible wall (3) over a large area.

13. Pump according to one of Claims 1 to 12, **characterized in that** the valves (8, 9) are located on the narrow sides of the wall (3) which correspond to the thickness.

14. Pump according to Claim 13, **characterized in that** the valves (8, 9) are arranged on narrow sides of the wall (3) which lie opposite one another.

15. Pump according to Claim 12, **characterized in that** the valves (8, 9) are arranged in recesses (10) of the pressure elements (5', 5").

16. Pump according to Claim 15, **characterized in that** the valves (8, 9) are arranged in recesses (10) of one of the pressure elements (5', 5").

17. Use of a pump according to one of Claims 1 to 16 as part of a prosthesis (11) for a lower extremity.

18. Use of a pump according to Claim 17, in which the force occurring due to body weight when a patient treads on the ground is utilized as the first force (F).

19. Use according to Claim 17 or 18 for the vacuum assistance of a suction socket of the prosthesis (11).

## Revendications

1. Pompe réalisée par une paroi (3 ; 21, 22 ; 31, 32, 33) formant un volume de fluide (1) fermé qui est mobile au moyen d'une première force (F) dans une direction d'une réduction de volume et au moyen d'une deuxième force dans une direction d'une augmentation de volume après une précédente réduction de volume, par une soupape d'entrée (8) qui communique avec une ouverture d'entrée (6) et par une soupape de sortie (9) réalisée dans une conduite de sortie (7) du volume de fluide (1), en tant que partie d'un système dans lequel sont engendrées des forces, en particulier par le poids d'un corps, la pompe étant réalisée en tant que pompe sous dépression, dont le volume peut être réduit par la force engendrée dans le système en tant que première force à l'encontre d'un matériau élastiquement déformable (2 ; 21, 22, 33), le fluide sortant du volume de fluide (1) par la conduite de sortie (7) et le matériau élastiquement déformable (2 ; 21, 22, 33) étant formé par la paroi (3 ; 21, 22 ; 31, 32, 33) elle-même ou étant disposé à l'intérieur du volume de fluide (1) qui, par son déplacement vers sa position de rappel après la fin de la première force, réalise la deuxième force qui agit à l'encontre de la dépression engendrée.

2. Pompe selon la revendication 1, **caractérisée en ce que** la paroi présente deux parois rigides (31, 32) opposées l'une à l'autre et que le matériau élastiquement déformable (33) est disposé dans l'espace compris entre les parois.

3. Pompe selon la revendication 2, **caractérisée en ce que** le matériau élastiquement déformable est constitué par un insert étanche (33) avec un bord entourant et limitant le volume de fluide (1).

4. Pompe selon la revendication 1, **caractérisée en ce que** la paroi (3) est flexible et que le matériau déformable élastiquement est en contact avec la paroi flexible (3).

5. Pompe selon la revendication 4, **caractérisée en ce que** le matériau élastiquement déformable est un matériau perméable qui est disposé à l'intérieur du volume de fluide (1) dont la force de rappel constitue la deuxième force.

6. Pompe selon la revendication 5, **caractérisée en ce que** le matériau perméable est une mousse (2) à pores ouverts.

7. Pompe selon la revendication 6, **caractérisée en ce que** le matériau élastique remplit le volume de fluide (1).

8. Pompe selon la revendication 7, **caractérisée en ce que** la matière élastique est entourée de tous les côtés par la paroi flexible (3).

9. Pompe selon l'une des revendications 4 à 8, **caractérisée en ce que** le matériau élastique est réalisé avec un allongement plat et du fait de cet allongement plat avec une faible épaisseur.

10. Pompe selon l'une des revendications 1 à 9, **caractérisée en ce que** le matériau élastiquement déformable est constitué par deux couches (21, 22) posées l'une contre l'autre présentant des profilages (23, 24) dirigés l'un vers l'autre et définissant des espaces intermédiaires et que les espaces intermédiaires peuvent être réduits au moyen de la première force.

11. Pompe selon la revendication 10, **caractérisée en ce que** les espaces intermédiaires entre les deux couches (21, 22) peuvent être réduits à zéro au moyen de la première force externe.

12. Pompe selon l'une des revendications 4 à 11, **caractérisée par** au moins un élément de pression (4, 5, 5', 5") s'appuyant de façon plane contre la paroi flexible (3).

13. Pompe selon l'une des revendications 1 à 12, **caractérisée en ce que** les soupapes (8, 9) se trouvent sur les côtés étroits de l'épaisseur de la paroi (3).

14. Pompe selon la revendication 13, **caractérisée en ce que** les soupapes (8, 9) sont disposées sur les côtés étroits opposés de la paroi (3).

15. Pompe selon la revendication 12, **caractérisée en ce que** les soupapes (8, 9) sont disposées dans des évidements (10) des éléments de pression (5', 5").

16. pompe selon la revendication 15, **caractérisée en ce que** les soupapes (8, 9) sont disposées dans des évidements (10) d'un des éléments de pression (5', 5").

17. Utilisation d'une pompe selon l'une des revendications 1 à 16 en tant que partie d'une prothèse pour une extrémité inférieure.

18. Utilisation d'une pompe selon la revendication 17 dans laquelle la force engendrée par le poids du corps est utilisée en tant que première force (F).

19. Utilisation selon la revendication 17 ou 18, pour assister le vide d'un fût d'aspiration de la prothèse (11).
